# EUROPÄISCHE PATENTSCHRIFT

(11) **EP 0 914 823 B1**
(45) Veröffentlichungstag und Bekanntmachung des Hinweises auf die Patenterteilung: **08.12.2004**
(21) Anmeldenummer: 98890326.6
(22) Anmeldetag: 05.11.1998
(51) Int. Cl.: A61K 31/35, A61K 9/22, A61K 9/36

(54) **Orale Retard-Präparation enthaltend Tramadol sowie Verfahren zu ihrer Herstellung**
Sustained release preparation comprising tramadol and a preparation for its production
Préparation orale à effet retard comprenant Tramadol et son procédé de fabrication

(30) Priorität: 06.11.1997 AT 187597
(43) Veröffentlichungstag der Anmeldung: 12.05.1999
(73) Patentinhaber: Lannacher Heilmittel Ges.m.b.H., A-8502 Lannach (AT)
(72) Erfinder: Reiter, Franz Josef, Mag.Dr.pharm., 8045 Graz (AT); Frisch, Thomas, 8054 Graz (AT); Posch, Werner Dr., 8071 Vasoldsberg (AT)
(74) Vertreter: Haffner, Thomas M.

(56) Entgegenhaltungen:
- EP-A- 0 682 945
- DE-A- 4 315 525

## Beschreibung

Die Erfindung bezieht sich auf eine orale Retard-Präparation in Form von Tabletten oder Filmtabletten mit verzögerter Wirkstoffabgabe sowie ein Verfahren zu ihrer Herstellung.

Matrixfilmtabletten zur peroralen Anwendung, welche Wirkstoffe in einer Weise enthalten, daß dieser nach Einnahme so stark verzögert aus der Tablettenmatrix freigesetzt wird, daß eine einmal tägliche Einnahme ausreicht, um therapeutisch relevante Blutplasmakonzentrationen aufrechtzuerhalten, sind prinzipiell bekannt.

Tramadolhydrochlorid-(RR,SS)-2-(Dimethylaminomethyl)-1-(3-methoxyphenyl)-cyclohexanol hydrochlorid - ist nach dem WHO-Stufenplan ein mittelstarkes Schmerzmittel der Stufe 2 und wird seit vielen Jahren erfolgreich in der Therapie von Schmerzen eingesetzt. Bislang befinden sich am Markt nur pharmazeutische Zubereitungen, die diesen Wirkstoff einer therapeutischen Anwendung unretardiert in Form von Tropfen, Ampullen, Zäpfchen, Kapseln etc. zugänglich machen, was für den Patienten eine mehrmals tägliche Verabreichung bzw. Einnahme bedeutet, um eine ausreichende Schmerzstillung aufrechtzuerhalten.

Vor kurzer Zeit erfolgte auch die Markteinführung einer retardierten Tramadol HCl Arzneiform (Filmtabletten) in drei verschiedenen Wirkstärken (100, 150 und 200 mg), die zur zweimal täglichen Einnahme geeignet ist. Die entsprechende Präparation ist beispielsweise in der EP-A2 642 788 beschrieben. Bei dieser bekannten für die zweimal tägliche Verabreichung geeigneten Darreichungsform wird ein feuchtigkeitsunempfindliches, physiologisch verträgliches Tramadolsalz (Tramadol HCl) mit mindestens einem Celluloseether und/oder Celluloseester, der in einer 2 gew.%igen wäßrigen Lösung bei 20° C eine Viskosität zwischen 3000 und 150000 mPas aufweist, als Matrixbildner verwendet.

Das Trägermaterial ist in den angeführten Beispielen zu 14 bis 34 Gew.% enthalten und bewirkt, daß in vitro nach 5 Stunden etwa 69 bis 89 % und längstens nach 12 Stunden 100 % des in der Tablette enthaltenen Wirkstoffes freigesetzt wird. Das bedeutet, daß mit dieser Formulierung die Freisetzung des genannten, gut wasserlöslichen Wirkstoffes nicht lange genug verzögert werden kann, um für eine 1 x tägliche Einnahme einen ausreichend hohen, analgetisch wirksamen Blutplasmaspiegel aufrechtzuerhalten.

Überraschenderweise wurde nun gefunden, daß bei Wahl einer geeigneten Matrix und bei Wahl einer geeigneten Zusammensetzung die Formulierung so gestaltet werden kann, daß tatsächlich mit nur einmal täglicher Verabreichung das Auslangen gefunden werden kann. Die Erfindung zielt nun darauf ab, eine derartige Formulierung bereit zu stellen, mit welcher der Wirkstoff Tramadolhydrochlorid bei einmaliger täglicher Darreichung einen ausreichend hohen analgetisch wirksamen Blutplasmaspiegel aufrechtzuerhalten erlaubt. Zur Lösung dieser Aufgabe besteht die erfindungsgemäße orale Retard-Präparation im wesentlichen darin, daß der Matrixbildner von in einem Lösungsmittel gelösten n-Hexadecanol (Cetylalkohol) gebildet ist und in einer Menge auf den Wirkstoff durch Aufsprühen aufgebracht ist, welche 20 bis 60 Gew.% des Tablettengewichtes entspricht, wobei als Wirkstoff Tramadolhydrochlorid in einer Menge von 15 bis 80 Gew.% eingesetzt ist und als Rest Tablettenhilfsstoffe und Füllstoffe, wie z.B. mikrokristalline Cellulose, Milchzucker, Gleit-, Schmier- und Fließregulierungsmittel, wie z.B. hochdisperses Siliziumdioxid, Magnesiumstearat oder Talk in Mengen von 0 bis 65 Gew.% eingesetzt ist. Dadurch, daß nun n-Hexadecanol als Matrixbildner verwendet wurde, wurde nun überraschenderweise gefunden, daß gegenüber dem bekannten Celluloseether- und/oder Celluloseester-matrizes eine deutliche Vergleichmäßigung der Freisetzung über 24 Stunden erzielt werden kann und daß tatsächlich gleichzeitig nicht nur die Freisetzung über 24 Stunden vergleichmäßigt werden konnte, sondern daß auch über diesen gesamten Zeitraum eine hinreichend hohe Plasmakonzentration aufrechterhalten werden kann. Die wasserunlösliche Cetylalkoholmatrix hat hiebei darüberhinaus den Vorzug, die Freisetzung weitestgehend unabhängig vom pH-Wert in über 24 Stunden ausreichender und gleichmäßiger Weise sicherzustellen. Gleichzeitig hat diese Cetylalkoholmatrix gegenüber bekannten Zubereitungen den Vorteil, daß der Wirkstoff unmittelbar in die Matrix eingearbeitet werden kann und in der Folge eine Filmtablette hergestellt werden kann, wodurch sich die Herstellung der Zubereitung wesentlich vereinfacht. Die Filmabdeckung dient hiebei in erster Linie der Geschmacksabdeckung.

Mit Vorteil ist die erfindungsgemäße Formulierung hiebei so getroffen, daß der Wirkstoff in Mengen von 25 bis 45 Gew.%, der Matrixbildner in Mengen von 25 bis 55 Gew.% und Tablettenhilfsstoffe in Mengen von 0,5 bis 40 Gew.% vorliegen, wobei vorzugsweise der Wirkstoff in Mengen zwischen 50 und 800 mg, vorzugsweise 200 bis 400 mg, je Tablette vorliegt.

Wie bereits eingangs erwähnt, hat die Verwendung von Cetylalkohol zur Folge, daß die Wirkstofffreisetzung pH-unabhängig und gleichmäßig über einen hinreichend langen Zeitraum gewährleistet ist, welcher mit bisherigen Formulierungen nicht ohne weiteres erzielt werden konnte. Vergleichsversuche mit Celluloseether und Celluloseestern sowie Eudragit haben durchwegs bestätigt, daß mit diesen bekannten Matrixbildnern das erfindungsgemäße Ziel, eine einmal tägliche Einnahme bei gleichzeitig ausreichend hohem analgetisch wirksamen Blutplasmaspiegel aufrechtzuerhalten, nicht gelingt.

Mit Rücksicht auf den bitteren Geschmack von Tramadolhydrochlorid ist, wie bereits zuvor erwähnt, eine Geschmacksabdeckung wünschenswert. Mit Vorteil ist die Ausbildung hiebei so getroffen, daß die Tabletten mit einem geschmacksabdeckenden wasserlöslichen Filmüberzug, wie z.B. Hydroxypropylmethylcellulose in Kombination mit Poly(meth)acrylaten, Polyethylenglykolen, Talk und Farbpigmenten, versehen sind.

Im Rahmen der erfindungsgemäßen Formulierung sind diejenigen Formulierungen besonders bevorzugt, bei welchen die Tabletten eine in vitro Wirkstoff-Freisetzung von 5 bis 35 Gew.% nach 1 h, 15 bis 45 Gew.% nach 2 h, 35 bis 75 Gew.% nach 6 h, 60 bis 90 Gew.% nach 12 h, 70 bis 98 Gew.% nach 18 h und 80 bis 100 Gew.% nach 24 h aufweisen.

Trotz der üblicherweise in hohem Maße vom pH-Wert abhängigen Freisetzungsgeschwindigkeit von Wirkstoffen gelingt es mit der Wahl von Cetylalkohol bzw. n-Hexadecanol eine Matrix zu schaffen, bei welchem die Freisetzungsgeschwindigkeit weitestgehend vom pH-Wert unabhängig wird. Der pH-Wert schwankt während der Passage eines Arzneimittels durch den Verdauungstrakt z.B. in Abhängigkeit von der Nahrungsaufnahme und kann Werte zwischen 1 und 7,5 erreichen. Für die gewünschte Langzeit Retardwirkung über einen Zeitraum von 24 Stunden ist daher die Auffindung einer geeigneten Matrix, bei welcher die Abgabe des Wirkstoffes aus der Formulierung im Gastrointestinaltrakt unabhängig vom jeweils vorherrschenden pH-Milieu erfolgt von besonderer Bedeutung.

Das erfindungsgemäße Verfahren zur Herstellung dieser oralen Retard-Präparation ist dadurch gekennzeichnet, daß der Wirkstoff mit mikrokristalliner Cellulose vermischt und mit der in Isopropanol gelösten Cetylalkoholmatrix besprüht wird, worauf das erhaltene fließfähige Granulat zu Tabletten verpreßt wird und anschließend der Filmüberzug aufgebracht wird, wodurch sich eine besondere einfache Herstellung ergibt. Naturgemäß kann anstelle des Cetylalkohols auch ein anderer Fettalkohol verwendet werden.

Die Erfindung wird nachfolgend anhand von Ausführungsbeispielen näher erläutert.

### Beispiel 1

Matrixtabletten entsprechend nachfolgender Rezeptur pro Filmtablette

| **Tablettenkern**: | |
|---|---|
| Tramadolhydrochlorid | 300,0 mg |
| Cetylalkohol | 345,5 mg |
| Mikrokristalline Cellulose | 250,0 mg |
| Magnesiumstearat | 4,5 mg |

| **Filmhülle:** | |
|---|---|
| Macrogol 6000 | 3,286 mg |
| Hydroxypropylmethylcellulose | 2,738 mg |
| Titandioxid | 3,296 mg |
| Talk | 9,858 mg |
| Poly(ethylacrylat,methyl- | 0,822 mg |
| methacrylat)2:1 | |

wurden in einer Ansatzgröße von 2000 Filmtabletten in folgender Weise produziert:

Der Wirkstoff wurde in einem Aeromatic-Strea 1-Wirbelschichtgranulator zusammen mit der mikrokristallinen Cellulose vorgemischt und mit einer isopropanolischen Lösung des Cetylalkohols besprüht, sodaß nach dem Sieben durch ein 1,25 mm Sieb ein homogenes, gut fließfähiges Granulat erhalten wurde.

Dieses wurde in einem Turbulamischer mit dem Magnesiumstearat 5 Minuten gemischt und anschließend auf einer Fette Rundlauftablettenpresse mit Oblong-Stempeln im Format 21 x 7,5 mm zu Tabletten mit einem Gewicht von 900 mg je Tablette verpreßt.

Die Befilmung erfolgte durch Aufsprühen einer wäßrigen Suspension in der oben angeführten Zusammensetzung nach dem in der Pharmaindustrie üblichen Dragierkesselverfahren.

Die in vitro Freisetzung des Wirkstoffes Tramadolhydrochlorid aus der Matrixtablette wurde nach Ph.Eur. 3^{rd} ed. 1997 mittels Blattrührerapparatur bestimmt. Die Temperatur des Lösungsmediums betrug 37 ± 0,5° C und die Rotationsgeschwindigkeit des Rührers 100 Umdrehungen pro Minute. Als Prüfmedien dienten wahlweise
a.) 1000 ml 0,1 N Salzsäure, pH = ca.1,2
b.) 750 ml 0,1 N Salzsäure, pH = ca.1,2 während der ersten beiden Stunden, ab der dritten Stunde Zusatz von 250 ml 0,2 M Na₃PO₄-lösung mit pH-Einstellung auf 6,8
c.) 1000 ml Phosphat-Citrat Puffer, pH = 6,8 nach Ph.Eur.

Die zum jeweiligen Zeitpunkt aus der Matrixtablette freigesetzte, im Prüfmedium gelöste Wirkstoffmenge wurde mittels Spektralfotometrie bestimmt. Es wurden die in nachfolgender Tabelle angeführten Freisetzungswerte (Mittelwerte aus n = 6) gefunden:

| Zeit (Stunden) | Kumulativer freigesetzter Tramadolhydrochloridanteil in Gew.% | | |
|---|---|---|---|
| | Prüfmedium a.) | Prüfmedium b.) | Prüfmedium c.) |
| 1 | 21 | 20 | 19 |
| 2 | 31 | 29 | 29 |
| 4 | 44 | 41 | 45 |
| 6 | 55 | 51 | 55 |
| 8 | 65 | 58 | 64 |
| 12 | 80 | 72,5 | 76 |
| 18 | 93 | 87 | 89 |
| 24 | 100 | 100 | 100 |

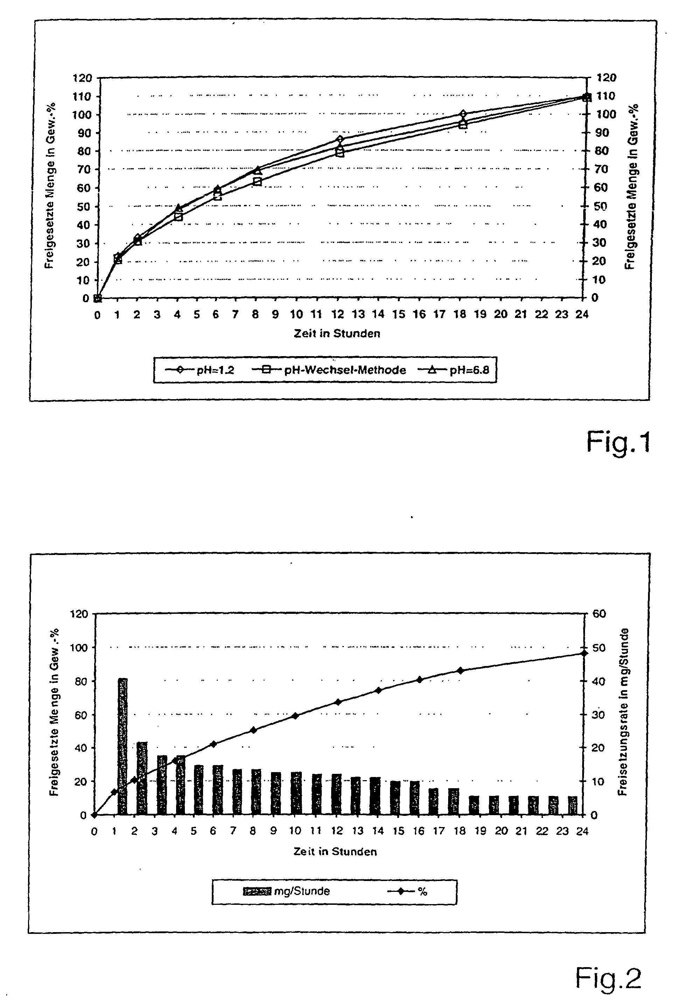

Die entsprechenden in vitro Freisetzungsprofile der Tramadolhydrochlorid retard 300 mg 1 x täglich Filmtabletten sind in Fig. 1 bei unterschiedlichen ph-Werten abgebildet.

### Beispiel 2

Matrixtabletten entsprechend der Rezeptur von Beispiel 1 wurden in einer Ansatzgröße von 2000 Filmtabletten in folgender Weise produziert:
Der Wirkstoff wurde in einem Aeromatic-Strea 1-Wirbelschichtgranulator vorgewärmt und mit einer isopropanolischen Lösung des Cetylalkohols besprüht, sodaß nach dem Sieben durch ein 1,25 mm Sieb ein homogenes, gut fließfähiges Granulat erhalten wurde.

Dieses wurde in einem Turbulamischer mit der mikrokristallinen Cellulose sowie mit dem Magnesiumstearat 15 Minuten gemischt und anschließend auf einer Fette Rundlauftablettenpresse mit Oblong-Stempeln im Format 21 x 7,5 mm zu Tabletten mit einem Gewicht von 900 mg je Tablette verpreßt.

Durch das direkte Besprühen des Wirkstoffes mit dem hydrophoben Matrixbildner Cetylalkohol konnte der Retardierungseffekt im Vergleich zu Beispiel 1 noch verstärkt werden.

Die in vitro Freisetzung des Wirkstoffes wurde gemäß Beispiel 1 untersucht. Es wurden die in nachfolgender Tabelle angeführten Freisetzungswerte (Mittelwerte aus n = 6) gefunden:

| Zeit (Stunden) | Kumulativer freigesetzter Tramadolhydrochloridanteil in Gew.% | Freigesetzter Tramadolhydrochloridanteil pro Zeit (Freisetzungsrate) in mg/Stunde |
|---|---|---|
| | Prüfmedium c.) | Prüfmedium c.) |
| 1 | 13,5 | 40,4 |
| 2 | 20,6 | 21,5 |
| 4 | 32,1 | 17,3 |
| 6 | 41,7 | 14,4 |
| 8 | 50,5 | 13,2 |
| 10 | 58,8 | 12,4 |
| 12 | 66,7 | 11,8 |
| 14 | 74,0 | 11,0 |
| 16 | 80,6 | 9,9 |
| 18 | 85,8 | 7,8 |
| 24 | 96,5 | 5,4 |

Das entsprechende kumulative in vitro Freisetzungsprofil sowie die dazugehörigen Freisetzungsraten der Tramadolhydrochlorid retard 300 mg 1 x täglich Filmtabletten aus Beispiel 2 sind in Fig. 2 abgebildet.

### Pharmakokinetische Daten zu Tramadolhydrochlorid retard 1 x täglich Filmtabletten gemäß Beispiel 2

Die dargestellte Erfindung zeichnet sich durch eine geringe pH-Abhängigkeit des Freisetzungsverhaltens aus. Das wird durch den im Beispiel 1 ausgeführten Vergleich des Freisetzungsverhaltens bei Prüfmedien unterschiedlichen pH's verdeutlicht. Dies ist insbesondere für langsam freisetzende Zubereitungen wichtig, da während der Magen-Darm-Passage die Zubereitung schon natürlicherweise unterschiedlichen pH-Werten ausgesetzt ist. Mahlzeit- und krankheitsbedingte pH-Veränderungen im Magen-Darm-Milieu könnten die Aufnahme in den Körper und damit die Wirksamkeit so zusätzlich beeinträchtigen.

Für den Anspruch einer Zubereitung, über 24 h nach Einnahme, wirksame Plasmakonzentrationen mit hinreichender Sicherheit zu liefern, ist es nach Literaturangaben erforderlich, daß die Plasmakonzentrationen, die 24 h nach einmaliger Gabe gefunden werden, höher als 100 ng/ml sind.

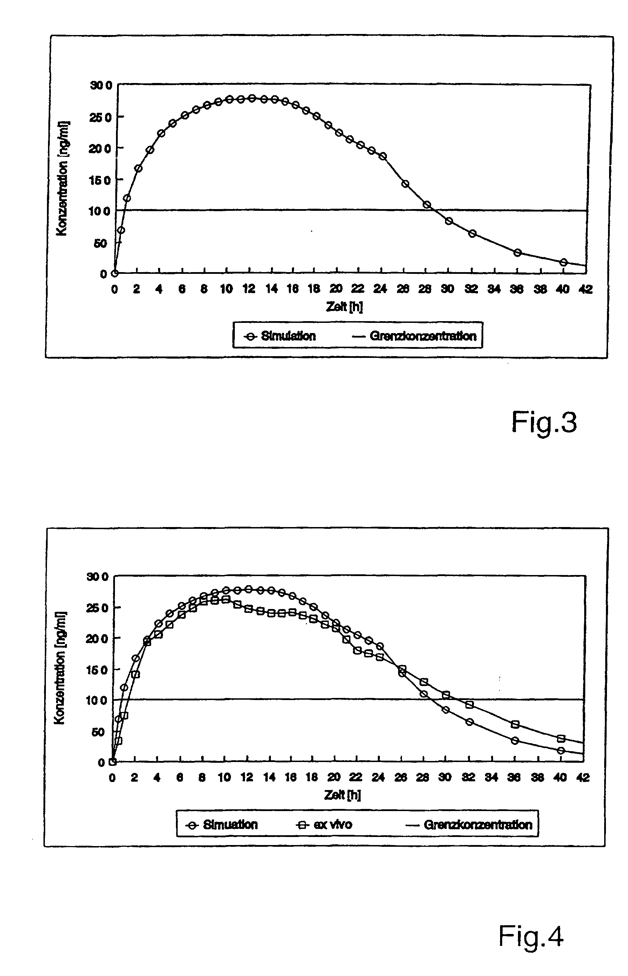

Um die Vorhersagbarkeit der Wirkdauer zu bestimmen, wurde mit einer pharmakokinetischen Simulation aus den Freisetzungsdaten des Beispiels 2 mit Hilfe eines offenen Ein-Kompartiment-Modells der Verlauf der vermutlichen Plasmakonzentrationen berechnet. Hierzu wurden die mittleren Freisetzungsdaten des Beispiels 2 als Absorptionswerte über die Zeit, ein Verteilungsvolumen von 306 Litern und eine Eliminationshalbwertszeit von 5.84 Stunden, entsprechend einer Eliminationskonstante von 0.1185 [1/h] angenommen. Das einfache Simulationsverfahren ist bei Fachleuten bekannt, die angenommenen Werte für das Verteilungsvolumen und die Eliminationshalbwertszeit sind Mittelwerte der verfügbaren Literatur. Die Simulation führt zu dem in Fig. 3 dargestellten Plasmakonzentrationszeitverlauf. Es ist erkennbar, daß die wirksame Konzentration erst nach ca. 28 Stunden unterschritten wird.

Um diesen hypothetischen Konzentrationszeitverlauf zu überprüfen, wurden 6 Probanden jeweils eine Zubereitung des Beispiels 2 im nüchternen Zustand verabreicht und die Plasmakonzentration über 48 h danach bestimmt.

| Zeit (Stunden) | Tramadolserumkonzentration | |
|---|---|---|
| | Mittelwert | Standardabweichung |
| 0 | 0,0 | 0,0 |
| 0,5 | 33,6 | 20,6 |
| 1 | 73,4 | 14,9 |
| 2 | 140,1 | 40,5 |
| 3 | 192,4 | 52,7 |
| 4 | 205,7 | 59,5 |
| 5 | 221,8 | 61,8 |
| 6 | 237,9 | 78,8 |
| 7 | 248,1 | 78,2 |
| 8 | 258,3 | 79,9 |
| 9 | 259,5 | 82,6 |
| 10 | 260,7 | 88,6 |
| 11 | 253,4 | 84,4 |
| 12 | 246,2 | 81,4 |
| 13 | 242,4 | 73,6 |
| 14 | 238,6 | 69,5 |
| 15 | 239,2 | 75,8 |
| 16 | 239,8 | 82,5 |
| 17 | 235,0 | 73,8 |
| 18 | 230,1 | 66,3 |
| 19 | 221,9 | 59,4 |
| 20 | 213,7 | 56,8 |
| 21 | 196,7 | 51,4 |
| 22 | 179,7 | 50,8 |
| 23 | 173,9 | 47,4 |
| 24 | 168,1 | 45,1 |
| 26 | 149,9 | 45,5 |
| 28 | 128,6 | 41,3 |
| 30 | 108,2 | 36,7 |
| 32 | 91,5 | 23,9 |
| 36 | 59,7 | 11,3 |
| 40 | 37,5 | 11,3 |
| 48 | 10,9 | 9,7 |

24 h nach Einnahme betrug die mittlere Tramadolkonzentration 168.1 +/- 45.1 (Mittelwert +/- Standardabweichung) und war somit deutlich oberhalb der zur Wirkung notwendigen Konzentration. Fig. 4 zeigt die hypothetische, simulierte Tramadolplasmakonzentrationszeitkurve zusammen mit der gefundenen Mittelwertskurve.

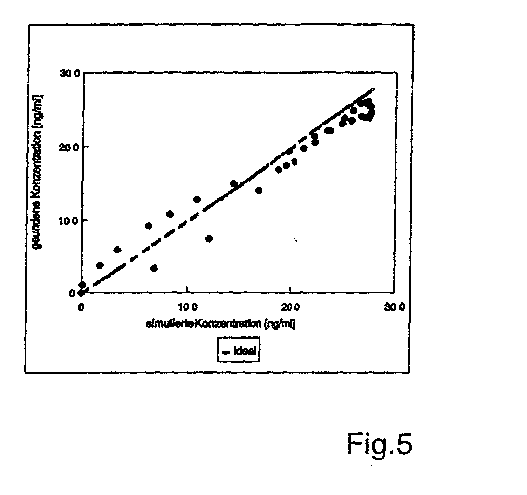

Die gefundene Tramadolkonzentration Fig. 5 korreliert mit der durch Simulation vorhergesagten mit einem Bestimmtheitsmaß von r2=0.86. Die überraschend gute Vorhersagbarkeit kann als ein besonderes Qualitätsmerkmal dieser Zubereitung angesehen werden.

## Patentansprüche

1. Orale Retard-Präparation in Form von Tabletten oder Filmtabletten mit verzögerter Wirkstoffabgabe, **dadurch gekennzeichnet, dass** der Matrixbildner von in einem Lösungsmittel gelösten n-Hexadecanol (Cetylalkohol) gebildet ist und in einer Menge auf den Wirkstoff durch Aufsprühen aufgebracht ist, welche 20 bis 60 Gew.% des Tablettengewichtes entspricht, wobei als Wirkstoff Tramadolhydrochlorid in einer Menge von 15 bis 80 Gew.% eingesetzt ist und als Rest Tablettenhilfsstoffe und Füllstoffe, wie z.B. mikrokristalline Cellulose, Milchzucker, Gleit-, Schmier- und Fließregulierungsmittel, wie z.B. hochdisperses Siliziumdioxid, Magnesiumstearat oder Talk in Mengen von 0 bis 65 Gew.% eingesetzt ist.

2. Orale Retard-Präparation nach Anspruch 1, **dadurch gekennzeichnet, dass** der Wirkstoff in Mengen von 25 bis 45 Gew.%, der Matrixbildner in Mengen von 25 bis 55 Gew.% und Tablettenhilfsstoffe in Mengen von 0,5 bis 40 Gew.% vorliegen.

3. Orale Retard-Präparation nach Anspruch 1 oder 2, **dadurch gekennzeichnet, dass** der Wirkstoff in Mengen zwischen 50 und 800 mg, vorzugsweise 200 bis 400 mg, je Tablette vorliegt.

4. Orale Retard-Präparation nach Anspruch 1, 2 oder 3, **dadurch gekennzeichnet, dass** die Tabletten mit einem geschmacksabdeckenden wasserlöslichen Filmüberzug, wie z.B. Hydroxypropylmethylcellulose in Kombination mit Poly(meth)acrylaten,Polyethylenglykolen, Talk und Farbpigmenten, versehen sind.

5. Orale Retard-Präparation nach einem der Ansprüche 1 bis 4, **dadurch gekennzeichnet, dass** die Tabletten eine in vitro Wirkstoff-Freisetzung von 5 bis 35 Gew.% nach 1 h, 15 bis 45 Gew.% nach 2 h, 35 bis 75 Gew.% nach 6 h, 60 bis 90 Gew.% nach 12 h, 70 bis 98 Gew.% nach 18 h und 80 bis 100 Gew.% nach 24 h aufweisen.

6. Verfahren zur Herstellung einer oralen Retard-Präparation nach einem der Ansprüche 1 bis 5, **dadurch gekennzeichnet, dass** der Wirkstoff mit mikrokristalliner Cellulose vermischt und mit der in Isopropanol gelösten Cetylalkoholmatrix besprüht wird, worauf das erhaltene fließfähige Granulat zu Tabletten verpresst wird und anschließend der Filmüberzug aufgebracht wird.

## Claims

1. An oral sustained-release preparation in the form of tablets or film-coated tablets for the sustained release of an active component, **characterized in that** the matrix former is comprised of n-hexadecanol (cetyl alcohol) dissolved in a solvent and applied on the active component by spraying in an amount corresponding to 20 to 60 wt.% of the tablet weight, wherein as said active component tramadol hydrochloride is used in an amount ranging from 15 to 80 wt.%, the balance being tablet adjuvants and fillers such as, e.g., microcrystalline cellulose, milk sugar, lubricants and flow regulation agents such as, e.g., highly disperse silicon dioxide, magnesium stearate or talc in amounts ranging from 0 to 65 wt.%.

2. An oral sustained-release preparation according to claim 1, **characterized in that** said active component is present in an amount ranging from 25 to 45 wt.%, said matrix former is present in an amount ranging from 25 to 55 wt.%, and said tablet adjuvants are present in amounts ranging from 0.5 to 40 wt.%.

3. An oral sustained-release preparation according to claim 1 or 2, **characterized in that** said active component is present in an amount ranging between 50 and 800 mg, preferably 200 to 400 mg, per tablet.

4. An oral sustained-release preparation according to claim 1, 2 or 3, **characterized in that** said tablets are provided with a flavour-masking water-soluble film coating such as, e.g., hyroxypropylmethyl cellulose in combination with poly(meth)acrylates, polyethylene glycols, talc and colour pigments.

5. An oral sustained-release preparation according to any one of claims 1 to 4, **characterized in that** said tablets exhibit an in-vitro release of said active component ranging from 5 to 35 wt.% after 1 hr, 15 to 45 wt.% after 2 hrs, 35 to 75 wt.% after 6 hrs, 60 to 90 wt.% after 12 hrs, 70 to 98 wt.% after 18 hrs and 80 to 100 wt.% after 24 hrs.

6. A method for preparing an oral sustained-release preparation according to any one of claims 1 to 5, **characterized in that** said active component is mixed with microcrystalline cellulose and sprayed with the cetyl alcohol matrix dissolved in isopropanol, whereupon the free-flowing granules obtained are pressed into tablets and the film coating is applied subsequently.

## Revendications

1. Préparation orale à effet retard sous forme de comprimés ou de comprimés pelliculés permettant une délivrance de substance active retardée, **caractérisée en ce que** l'agent de formation de la matrice est formé à partir de n-hexadécanol (alcool cétylique) dissous dans un solvant et est déposé par atomisation sur la substance active en une quantité correspondant à 20-60 % en poids du poids des comprimés, du chlorhydrate de tramadol étant introduit en tant que substance active en une quantité allant de 15 à 80 % en poids et des auxiliaires de compression et des charges, par exemple de la cellulose microcristalline, du lactose, des agents antifriction, des lubrifiants et des fluidisants, par exemple du dioxyde de silicium hautement dispersé, du stéarate de magnésium ou du talc, étant introduits, en des quantités allant de 0 à 65 % en poids, pour former le complément.

2. Préparation orale à effet retard selon la revendication 1, **caractérisée en ce que** la substance active est présente en des quantités allant de 25 à 45 % en poids, **en ce que** l'agent de formation de la matrice est présent en des quantités allant de 25 à 55 % en poids et **en ce que** les auxiliaires de compression sont présents en des quantités allant de 0,5 à 40 % en poids.

3. Préparation orale à effet retard selon la revendication 1 ou 2, **caractérisée en ce que** la substance active est présente en des quantités comprises entre 50 et 800 mg, de préférence entre 200 et 400 mg par comprimé.

4. Préparation orale à effet retard selon la revendication 1, 2 ou 3, **caractérisée en ce que** les comprimés sont dotés d'un enrobage pelliculé hydrosoluble et masqueur de goût, comme l'hydroxypropylméthylcellulose combinée à des poly(méth)acrylates, des polyéthylène glycols, du talc et des pigments colorants.

5. Préparation orale à effet retard selon l'une des revendications 1 à 4, **caractérisée en ce que** les comprimés présentent une libération de la substance active *in vitro* allant de 5 à 35 % en poids au bout de 1 heure, de 15 à 45 % en poids au bout de 2 heures, de 35 à 75 % en poids au bout de 6 heures, de 60 à 90 % en poids au bout de 12 heures, de 70 à 98 % en poids au bout de 18 heures et de 80 à 100 % en poids au bout de 24 heures.

6. Procédé pour préparer une préparation orale à effet retard selon l'une des revendications 1 à 5, **caractérisé en ce que** la substance active est mélangée à de la cellulose microcristalline et atomisée avec la matrice d'alcool cétylique dissoute dans de l'isopropanol, puis le granulat fluide obtenu est compressé sous la forme de comprimés puis enfin on dépose l'enrobage pelliculé.
